(19) Europäisches Patentamt
European Patent Office
Office européen des brevets

(11) Publication number: **0 321 211 A1**

(12) **EUROPEAN PATENT APPLICATION**

(21) Application number: **88311828.3**

(22) Date of filing: **14.12.88**

(51) Int. Cl.4: **C07D 249/08 , A01N 43/653**

(30) Priority: **17.12.87 US 134025**

(43) Date of publication of application:
**21.06.89 Bulletin 89/25**

(84) Designated Contracting States:
**ES GR**

(71) Applicant: **E.I. DU PONT DE NEMOURS AND COMPANY**
**1007 Market Street**
**Wilmington Delaware 19898(US)**

(72) Inventor: **Basarab, Gregory Steven**
**705 W. 28th Street**
**Wilmington Delaware 19802(US)**
Inventor: **Greenberg, Richard Scot**
**41-10 Erli Road**
**Fairlawn New Jersey 07410(US)**

(74) Representative: **Hildyard, Edward Martin et al**
**Frank B. Dehn & Co. European Patent Attorneys Imperial House 15-19 Kingsway**
**London WC2B 6UZ(GB)**

(54) **Antifungal carbinols.**

(57) Antifungal carbinols having the formula

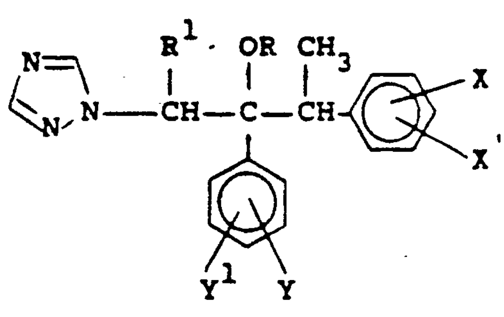

wherein:
R and $R^1$ are independently H or $CH_3$;
X and $Y^1$ are independently H, 1 to 3 halogens or $CF_3$; and
$X'$ and Y are independently H, $CH_3$, $CF_3$, $OCF_2H$, $OCH_3$ or $SCH_3$
and their agriculturally and pharmaceutically suitable salts are useful as plant disease control agents.

The compounds may be made e.g. by reacting an appropriate oxirane of formula:

EP 0 321 211 A1

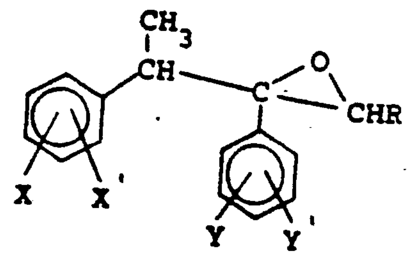

with triazole or its alkali metal salt.

# ANTIFUNGAL CARBINOLS

## Field of the Invention

This invention relates to antifungal carbinols, particularly α-methyl benzyl carbinols, compositions containing them, processes for preparing them and methods of using them as antifungal agents in plants and mammals.

## Background of the Invention

Plant pathogenic fungi and other disease incitants cause extensive losses in crops annually. While there are commercially available materials used to control many plant diseases, further improvement in this art is needed if full food and fiber production is to be realized. Systemic fungal infections are also of increasing importance because of continued and expanded use of immunosuppresssive therapies, antimicrobial therapies and indwelling catheters.

The following references are related to the instant invention:

German Patent 3,018,865, published May 16, 1980 discloses antimycotic agents of the formula:

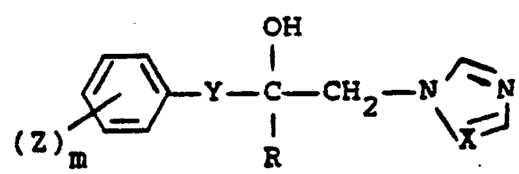

where amongst others
R is alkyl, optionally substituted cycloalkyl or optionally substituted phenyl radical;
X is N, or a CH group;
Y is $-OCH_2-$, $-CH_2CH_2-$ or $CH=CH$;
Z is halogen, alkyl, cycloalkyl, alkoxy, alkythio, etc.

German Patent 3,314,548-A, published April 21, 1983 discloses substituted 1-hydroxy-ethyl-triazole derivatives of the formula:

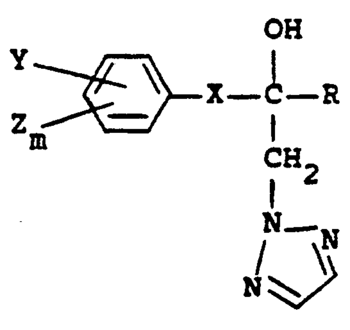

where amongst others
R is alkyl, cycloalkyl or phenyl optionally substituted;
X is $-OCH_2-$, $-SCH_2-$, $-(CH_2)_p$ or $-CH=CH-$;
Z is halogen, alkyl, alkoxy, alkylthio, haloalkyl, haloalkoxy, or haloalkylthio;
m and p are 0, 1 and 2.

The compounds are antimycotics for treating dermatophytomycoses and systemic mycoses caused, e.g., by Candida sp., Aspergillus sp. and Trichophyton sp.

B. Sugavanam in U.S. Patent 4,507,140 issued March 26, 1985 discloses fungicidal or plant growth regulating β-styryl triazoles or imidazoles,

amongst others of the formula:

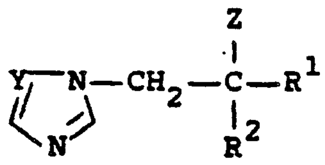

where
R' is CH=CH=X; -C≡C-X or $-CH_2-CH_2-X$;
X is substituted aryl, aralkyl, or heterocycle;
$R^2$ is alkyl, cycloalkyl, or optionally substituted aryl;
Z is $OR^3$;
$R^3$ is H, acetyl;
Y is -N- or -CH-.

European Patent 117,578-A, published February 23, 1984 discloses heterocyclic-hydroxy-alkyl alkyl ketone(s) and analogues of the formula:

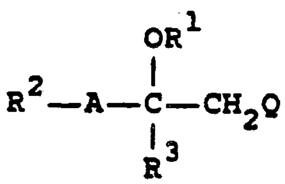

where
A is CO amongst others;
Q is imidazoyl or 1H- or 4H-1,2,4-triazol-1-yl;
R' is H, 1-5C alkyl, or 1-8C acyl;
$R^2$ and $R^3$ are 1-5C alkyl, 3-6C cycloalkyl, 1-6C alkenyl, benzyl (optionally substituted by 1-3 halogen), pyridyl, furyl, thienyl, or phenyl optionally substituted by 1-3 halogen, 1-3 alkyl, or 1-3C alkoxy.

Belgian Patent 900,594-A published September 22, 1983 discloses 1-phenyl-1-azolyl-hydroxyethyl cycloalkane derivatives of the formula:

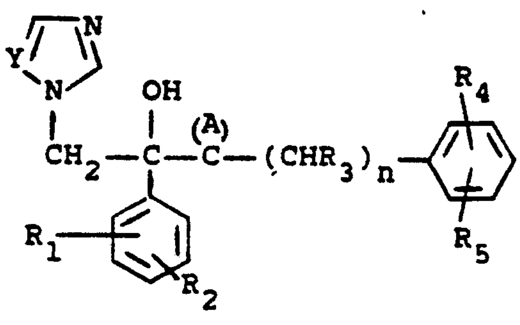

where
$R_1$ and $R_2$ = H, halo, $NO_2$, lower alkyl, alkenyl, alkoxy or alkylthio (all optionally substituted by 1 or more halo), or optionally substituted phenyl or phenoxy;
$R_3$ = H or lower alkyl;
$R_4$ and $R_5$ = H or halo;
Y = CH or N;
A = 2-7C alkylene;
n = 0 or 1.

U.S. Patent 4,358,458, published November 9, 1982 discloses 1-imadiazole 2-phenyl-3-phenyl-2-propanol derivatives of the formula:

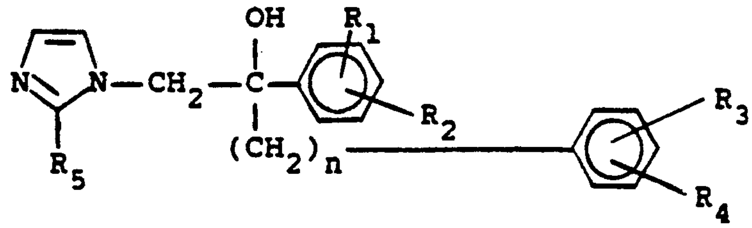

where

$R_1$, $R_2$, $R_3$ and $R_4$ are H, halogen, $C_1$-$C_4$ alkyl or $C_1$-$C_4$ alkoxy; and

n is 0 or 1.

EP-A 090,993, published October 12, 1983 discloses 1-azoyl-2-phenyl-3,3-dialkyl-2-propanol derivatives of the formula:

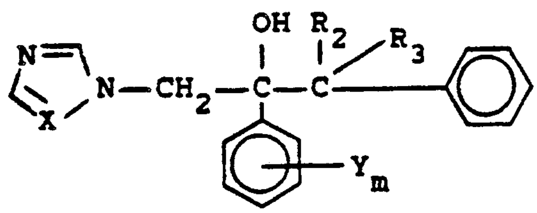

where

$R_2$ and $R_3$ are alkyl.

EP-A 15756, published September 17, 1980 discloses 1-azoyl-2,-phenyl-3-phenyl-2-propanol derivatives of the formula:

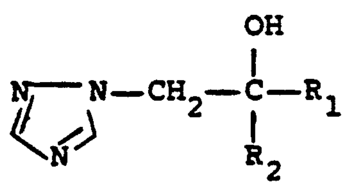

where

$R_1$ is alkyl, cycloalkyl or optionally substituted phenyl; and

$R_2$ is optionally substituted phenyl or benzyl.

U.S. Patent 4,414,210, published November 8, 1983 discloses 1-azoyl-2-phenyl-3-phenyl-2-propanol derivatives of the formula:

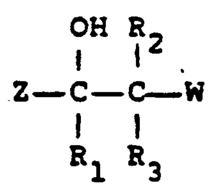

where

Z is an optionally substituted aryl;

$R_1$, $R_2$ and $R_3$ are in part independently a hydrogen, $C_1$-$C_2$ alkyl, optionally substituted aryl or optionally substituted $C_1$-$C_4$ arallyl; and

W is triazole.

## Summary of the Invention

This invention pertains to compounds of Formula I, including geometric and stereoisomers, suitable agricultural and pharmaceutical compositions containing them, including crop oil-based formulations and their use as agricultural and pharmaceutical fungicides.

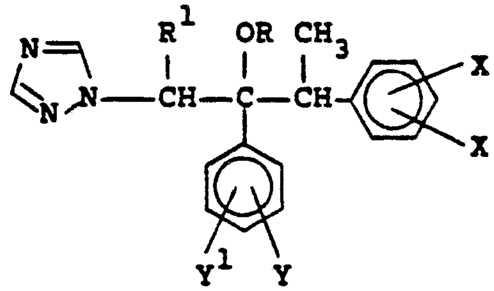

**Formula I**

wherein:
R and $R^1$ are H or $CH_3$;
X and $Y^1$ are independently H, 1 to 3 halogens e.g. F, Cl, Br and I or $CF_3$; and
X′ and Y are independently H, $CH_3$, $CF_3$, CN, $OCF_2H$, $OCH_3$ or $SCH_3$
and their agriculturally and pharmaceutically suitable salts.

This invention also relates to a method for controlling fungus diseases, particularly fungus diseases of living plants which comprises applying to the locus to be protected an effective amount of a compound of Formula I or its agriculturally suitable salts usually admixed with an agriculturally acceptable diluent or carrier.

Preferred for their high fungicidal activity and/or favorable ease of synthesis are compounds of Formula I wherein R and R′ are H.

More preferred compounds for their higher fungicidal activity and/or more favorable ease of synthesis are preferred compounds wherein X and $Y^1$ are independently 1 to 2 F, Cl or Br.

Most preferred compounds are the more preferred compound wherein X and $Y^1$ are independently 1 to 2 F; and Y and $X^1$ are H.

Specifically preferred because of their fungicidal activity and/or favorable ease of synthesis are the following compounds:

1-(1,2,4-triazol-1-yl)2-(4-fluorophenyl)-3-phenyl butane-2-ol;
1-(1,2,4-triazol-1-yl)2-(2,4-difluorophenyl)-3-phenylbutane-2-ol;
1-(1,2,4-triazol-1-yl)2-(4-fluorophenyl)-3-(4-fluorophenyl)butane-2-ol;
1-(1,2,4-triazol-1-yl)2-(2-fluorophenyl)-3-(4-fluorophenyl)butane-2-ol;
1-(1,2,4-triazol-1-yl)2-(2,4-difluorophenyl)-3-(4-fluorophenyl)butane-2-ol;
1-(1,2,4-triazol-1-yl)2-(2-chlorophenyl)-3-(4-fluorophenyl)butane-2-ol;
1-(1,2,4-triazol-1-yl)2-phenyl-3-(4-fluorophenyl)butane-2-ol;
1-(1,2,4-triazol-1-yl)2-(2,4-difluorophenyl)-3-(4-chlorophenyl)butane-2-ol;
1-(1,2,4-triazol-1-yl)2-(2,4-difluorophenyl)-3-(2-chlorophenyl)butane-2-ol;
1-(1,2,4-triazol-1-yl)2-(2-fluorophenyl)-3-(3-chlorophenyl)butane-2-ol;
1-(1,2,4-triazol-1-yl)2-(2,4-difluorophenyl)-3-(3-chlorophenyl)butane-2-ol;
1-(1,2,4-triazol-1-yl)2-(2,4-difluorophenyl)-3-(3-fluorophenyl)butane-2-ol;
1-(1,2,4-triazol-1-yl)2-(2-fluorophenyl)-3-(2,4-dichlorophenyl)butane-2-ol;
1-(1,2,4-triazol-1-yl)2-(2,4-difluorophenyl)-3-(2,4-dichlorophenyl)butane-2-ol;
1-(1,2,4-triazol-1-yl)2-(2,4-dichlorophenyl)-3-(2,4-dichlorophenyl)butane-2-ol;
1-(1,2,4-triazol-1-yl)2-(2-fluorophenyl)-3-(2,4-difluorophenyl)butane-2-ol;
1-(1,2,4-triazol-1-yl)2-(2,4-difluorophenyl)-3-(2,4-difluorophenyl)butane-2-ol;
1-(1,2,4-triazol-1-yl)2-(2,4-difluorophenyl)-3-(2,4-difluorophenyl)butane-2-ol;
1-(1,2,4-triazol-1-yl)2-(2,4-difluorophenyl)-3-(2-fluorophenyl)butane-2-ol; and
1-(1,2,4-triazol-1-yl)2-(4-chlorophenyl)-3-(2-fluorophenyl)butane-2-ol;
and their agriculturally and pharmaceutically suitable salts.

Detailed Description of the Invention

## Synthesis

The novel compounds of Formula (I) can be prepared using the reactions and techniques described in this section. The reactions are usually performed in a solvent appropriate to the reagents and materials employed, and suitable for the transformation being effected. In some cases functional groups on the starting materials may need to be protected by standard protecting groups reported in the chemical literature which are well known to one skilled in the art.

In some cases, substituents on the starting materials may be incompatible with some of the reaction conditions required in some of the methods described. Such restrictions to the substituents which are compatible with the reaction conditions will be readily apparent to one skilled in the art and alternative methods described must then be used.

The compounds of the present invention can contain at least one chiral center and as such can exist as two individual isomers or as a racemic mixture of both. This invention relates to the (S) isomer, as well as to racemic mixtures containing both isomers.

For the purposes of this invention, the (R)-isomer of compounds of Formula (I) is intended to mean compounds of the configuration depicted:

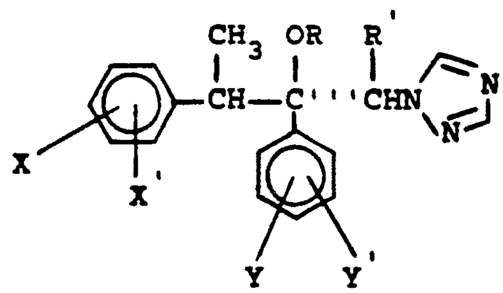

(R)-Ia

The compounds of Formula (I) can be prepared by reacting the corresponding olefinic compounds (II) with hydrogen in the presence of a suitable heterogeneous catalyst, such as palladium, platinum or rhodium on carbon, or a homogeneous catalyst, such as tris(triphenylphosphine)chlororhodium, as outlined in Scheme I. Typically, hydrogen pressures of 1 to 4 atm. and temperatures of 0° to 100° C can be used. In some cases, higher pressures and/or temperatures, and catalysts such as Raney nickel or copper chromite, may be necessary (see H.O. House, Modern Synthetic Reactions, 2nd ed.; Benjamin, Menlo Park, 1972, pp. 1-34). Suitable solvents include alcohols such as methanol or ethanol, and esters such as ethyl acetate. Transfer hydrogenation, wherein a compound such as cyclohexene is used as the hydrogen source, in the presence of catalysts and solvents such as those described above, can also be employed.

SCHEME I

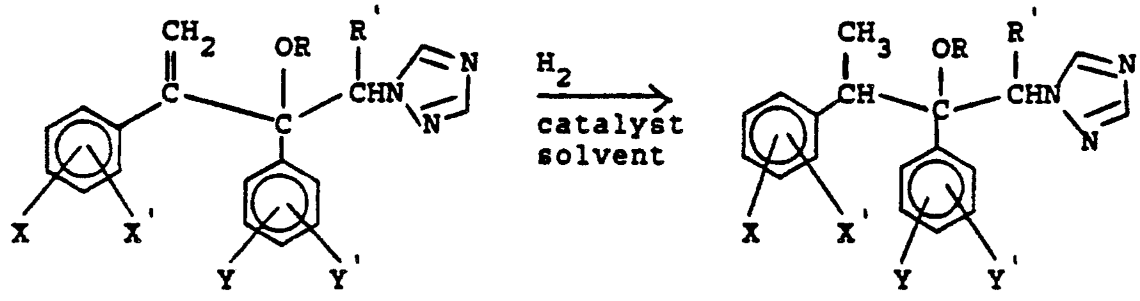


II I

Alternatively, compounds of Formula (I) may be prepared directly from the oxirane (III) or halohydrin (IV) and triazole or its alkali metal salt as outlined in Scheme II.

SCHEME II

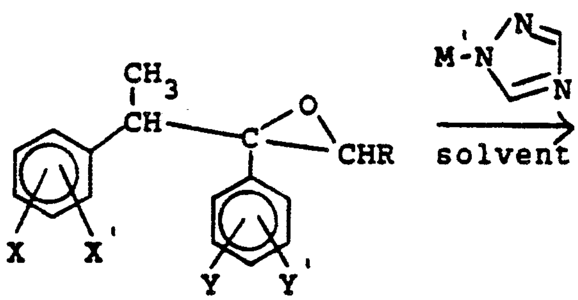


I

III

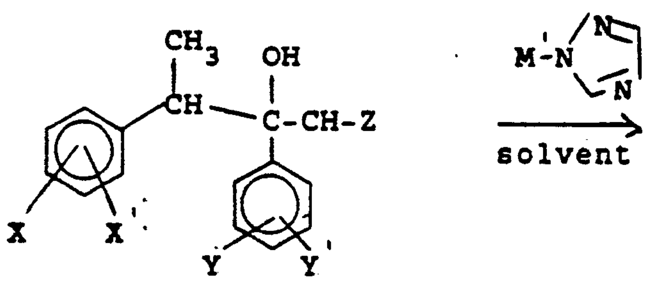


I

IV

Oxiranes (III) or halohydrins (IV), or a mixture thereof, may be produced by reacting an α-phenethyl organometallic compound (V), preferably a Grignard or organolithium reagent, with α-halo ketones (VI), in a suitable solvent such as diethyl ether or tetrahydrofuran as outlined in Scheme III.

SCHEME III

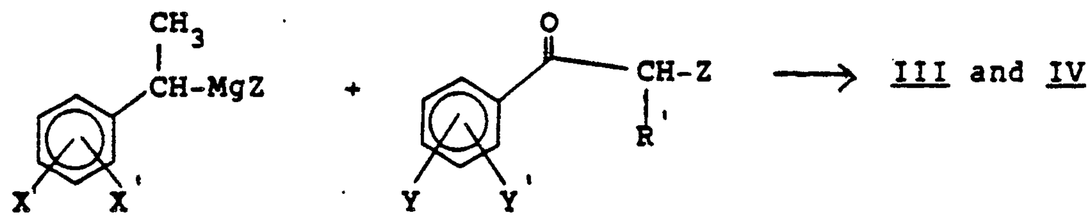


V VI

Compounds such as (II)(R = H) may be prepared by reacting an oxirane of Formula (VII) or a halohydrin of Formula (VIII), or a mixture of (VII) and (VIII), with 1,2,4-triazole or its alkali metal salt (preferably the $Na^+$ or $K^+$ salt) in a suitable solvent as outlined in Scheme IV.

SCHEME IV

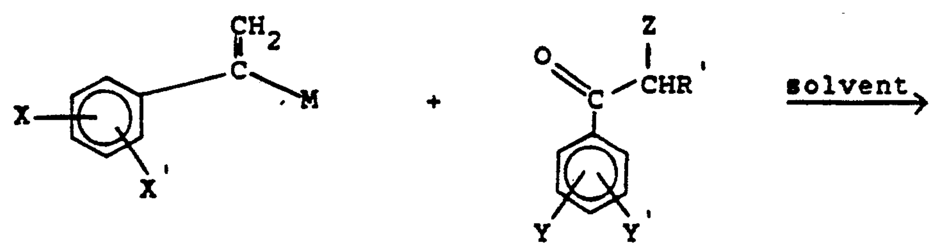


Va VIa

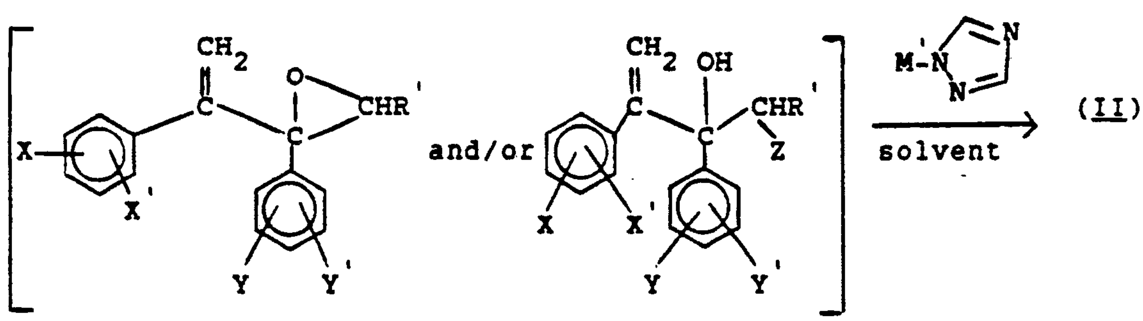


VII VIII

M = MgZ′ or Li;
Z and Z′ are (independently) I, Br, Cl; and
M′ = H or alkali metal.

When triazole is used, as in Scheme II and IV, an acid acceptor, such as potassium carbonate, sodium methoxide or sodium hydride, is added to the reaction mixture. Suitable inert solvents include polar, aprotic solvents such as dimethylformamide (DMF), dimethylsulfoxide (DMSO) and ethereal solvents such as tetrahydrofuran (THF). Non-polar solvents, such as toluene, may be used if a phase transfer catalyst, such as tetrabutylammonium bromide, is added. The reaction is carried out at a temperature in the range of 10° to 150° C, preferably from 50° to 120° C, for a period of 0.25 to 24 hours. It is recognized that varying amounts of the 4H-1,2,4-triazol-4-yl isomers of Formula (II) may be formed in the above reaction. The isomers can be separated, if desired, using standard separation techniques, e.g., chromatography.

The oxiranes of Formula (VII) may be prepared by allowing vinyl organometallic reagents, e.g., vinyl Grignard or vinyllithium reagents, of Formula (Va) to react with haloketones of Formula (VIa) in the presence of ethereal solvents, such as THF or diethyl ether, at a temperature ranging from -90° to 60° C, preferably -10° to 50° C, for 0.5 to 24 hours. Depending on the reaction conditions and the value of X in the haloketone starting material, the product may be an oxirane (VII), a halohydrin (VIII) or a mixture of (VII) and (VIII). If desired, the halohydrins (VIII) may be converted to oxiranes (VII) by treatment with base, e.g., potassium hydride (KH), in a solvent such as THF.

Oxiranes (VII) or halohydrins (VIII) can also be hydrogenated, under conditions such as those described above, to produce the reduced oxiranes (III) or halohydrins (IV). Treatment of (III) or (VIII), or a mixture thereof, with 1,2,4-triazole or its alkali metal salt, under conditions such as those described above, yields compounds of Formula (I) as outlined in Scheme V.

SCHEME V

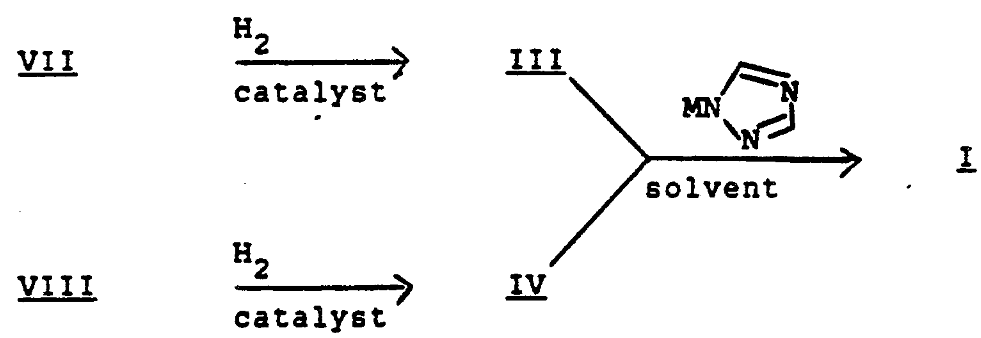


Compounds (I), (II), (IV) or (VIII) may be converted to the corresponding alkylated derivatives ($R = CH_3$) by a variety of known methods. (See T. W. Green, Protecting Groups in Organic Synthesis, John Wiley and Sons, New York, 1981.)

It will be noted by one skilled in the art that the compounds of Formulas (I), (II), (III), (IV), (VII) and (VIII) may be obtained as mixtures of diastereoisomers. These can, if desired, be separated in a conventional manner, e.g., by chromatography or fractional crystallization. It will be further noted that the compounds of Formulas (I), (II), (III), (IV), (VII) and (VIII) can exist as racemic mixtures of enantiomers. These can, if desired, be resolved using methods known to one skilled in the art.

The compounds of this invention and their preparation can be understood further by the following examples, but should not constitute a limitation thereof. In these examples, unless otherwise indicated, all temperatures are in degrees centigrade and parts and percentages are by weight.

Nucelar magnetic resonance (NMR) spectra were obtained in $CDCl_3$ solution, unless otherwise noted. Abbreviations for NMR spectra are s = singlet, d = doublet, t = triplet, q = quartet, m = multiplet; peak positions are reported as parts per million downfield from tetramethylsilane.

## Example 1

### 1-(1,2,4-Triazol-1-yl)-2-(2-Fluorophenyl)-3-(4-Fluorophenyl)-butane-2-ol

A solution of 5.0 g (15 mmol) of 1-(1,2,4-triazol-1-yl)-2-(2-fluorophenyl)-3-(4-fluorophenyl)-3-butan-2-ol in 30 ml of absolute ethanol was added to a stirred suspension of 0.50 g of 10% palladium on activated carbon in 120 ml of absolute ethanol, under hydrogen at 1 atmosphere pressure. The resulting mixture was stirred overnight at 20° C. The mixture was then filtered and concentrated, and the residue was purified by column chromatography on silica gel, eluting with 3:1 hexane/ethyl acetate followed by ethyl acetate, according to the procedure of: W. C. Still, M. Kahn and A. Mitra, J. Org. Chem., 1978, 43, 2923.

There was thus obtained 4.9 g (80.5%) of Isomer A as a white solid, m.p. 128-130° C, followed by 0.66 g (10.8%) of Isomer B as a white solid, m.p. 76-78° C.

NMR of Isomer A: δ
1.1 (d, 3H);
3.4 (q, 1H);
3.8 (1/2 of AB, 1H);
4.7 (s, 1H);
4.8 (1/2 of AB, 1H);
6.9-7.1 (m, 4H);
7.2 (m, 1H);
7.5 (m, 3H);
7.7 (s, 1H);
7.7 (2,1H).
NMR of Isomer B: δ
1.5 (d, 3H);
3.4 (q, 1H);
4.6 (1/2 of AB, 1H);
4.6 (s, 1H);
5.0 (1/2 of AB, 1H);
6.7 (m, 3H);
6.8-7.1 (m, 5H);
7.7 (s, 1H);
7.8 (s, 1H).

## Example 2

PART A: 2-(4-Fluorophenyl)-2-[1-(4-fluorophenyl)ethenyl] oxirane

PROCESS 1: Grignard Addition to an α-Haloketone

To a 25° C solution of Grignard reagent prepared from 6.0 g (0.030 mol) of 1-bromo-4′-fluorostyrene and 0.85 g (0.035 mol) of magnesium turnings in 60 mL of THF was added a solution of 5.2 g (0.030 mol) of 2-chloro-4′-fluoroacetophenone in 10 mL of THF. The solution was stirred for 2 hours at 25°. Saturated aqueous $NH_4Cl$ (10 mL) was added, the aqueous layer was extracted with 1:1 $Et_2O$/hexane and the combined organic layers were washed with brine, dried over $MgSO_4$ and evaporated to give 10.2 g of an amber oil. Analysis by NMR ($CDCl_3$) indicated that the desired oxirane was the major product : δ 3.1, 3.3 (two d, epoxide protons); 5.5, 5.8 (two s, vinyl protons). The material was of sufficient purity to be used in the next step.

PROCESS 2: Olefination of 2-(4-Fluorophenyl)-2-(4-fluorobenzoyl) oxirane

To a suspension of 4.3 g (0.012 mol) of methyltriphenylphosphonium bromide in 15 mL of THF cooled to -70° was added 8.4 mL (0.013 mol) of 1.55 M n-butyllithium over 3 min., keeping the temperature at less than -55°. The resulting yellow suspension was allowed to warm to 0° over 10 min. and was then treated with 2.6 g (0.010 mol) of 2-(4-fluorophenyl)-2-(4-fluorobenzoyl)oxirane in 5 mL of THF. The light-brown suspension was stirred for 6 hours at 25°. Standard workup gave 3.4 g of crude product which was flash chromatographed ($Et_2O$) to give 1.7 g of desired product, which was of sufficient purity to be used in the next step. NMR ($CDCl_3$) δ 3.1 (d): 3.3 (d); 5.5 (s); 5.8 (s).

PART B: 2,3-Bis (4-Fluorophenyl)-1-(1,2,4-triazol-1-yl)-3-buten-2-ol

A mixture of 10.2 g (0.030 mol) of crude 2-(4-fluorophenyl)-2-[1-(4-fluorophenyl)ethenyl]oxirane and 7.0 g (0.065 mol) of potassium triazole in 60 mL of DMF was heated at 60° overnight, then cooled and poured into 100 mL of 1:1 $Et_2O$/hexanes. After washing the organic layer three times with $H_2O$ and once with brine,

a precipitate formed in the organic layer. Filtering gave 4.8 g of a brown solid which was recrystallized from 500 mL of cyclohexane to yield 2.5 g of a light-tan powder, m.p. 136-137°:

NMR ($CDCl_3$) δ
1.7 (br s, OH);
4.7 (q, 2H);
5.3 (s, 1H);
5.5 (s, 1H);
6.8-7.1 (m, 6H);
7.4 (m, 1H);
7.8 (s, 1H);
7.9 (s, 1H);
IR (nujol) 3120 (br), 1900, 1600, 1505, 1220, 1139, 835 $cm^{-1}$.

PART C: 1-(1,2,4-Triazol-1-yl)-2,3-bis(4-fluorophenyl)-butan-2-ol

A solution of 2.5 g of the olefin from Part B in 150 mL of absolute ethanol was added to a stirred suspension of 0.25 g of 10% palladium on activated carbon in 50 mL of absolute ethanol, under hydrogen at 1 atmosphere pressure. The resulting mixture was stirred overnight at 20° C. The mixture was then filtered and concentrated, and the residue was purified by column chromatography on silica gel, eluting with 3:1 hexane/ethyl acetate followed by ethyl acetate, according to the procedure of: W. C. Still, M. Kahn and A Mitra, J. Org. Chem., 1978, 43, 2923.

There was thus obtained 2.06 g (76.%) of Isomer A as a white solid, m.p. 145-148° C, followed by 0.11 g (4%) of Isomer B as a white foam.

NMR (200 MHz) of Isomer A: δ
1.12 (d, J=7, 3H);
3.15 (g, J=7, 1H) ;
3.98 and 4.38 (AB, J=14, 2H);
4.41 (s, 1H);
6.9-7.1 (m, 4H);
7.3 (m, 2H);
7.4 (m, 2H);
7.56 (s, 1H);
7.74 (s, 1H).

NMR of Isomer B: δ
1.38 (d, J=7, 3H);
3.25 (q, J=7, 1H);
4.57 (s, 2H);
4.87 (s, 1H);
6.75-6.95 (m, 6H);
7.0 (m, 2H);
7.63 (s, 1H);
7.72 (s, 1H).

TABLE 1

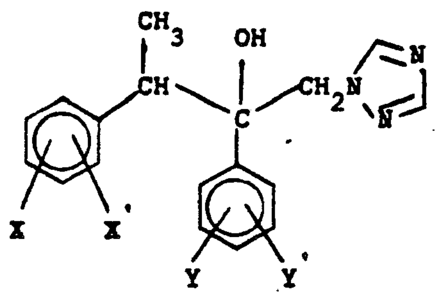

(I)

| EX. NO. | X | X' | Y' | Y | (m.p.°C) |
|---|---|---|---|---|---|
| 1 | H | H | H | H | |
| 2 | H | H | 2-F | H | |
| 3 | H | H | 4-F | H | |
| 4 | H | H | 2,4-di-F | H | 95-97 |
| 5 | H | H | 2-Cl | H | |
| 6 | H | H | 4-Cl | H | 100.5-102 |
| 7 | H | H | 2,4-di-Cl | H | |
| 8 | H | H | 2-Br | H | |
| 9 | H | H | 4-Br | H | |
| 10 | H | H | 2,4-di-Br | H | |
| 11 | H | H | 3-F | H | |
| 12 | H | H | 3-Cl | H | |
| 13 | H | H | 3-Br | H | |
| 14 | H | H | 2-$CF_3$ | H | |
| 15 | H | H | 3-$CF_3$ | H | |
| 16 | H | H | 4-$CF_3$ | H | |
| 17 | H | H | 2,4,6-tri-F | H | |
| 18 | H | H | 2,4,6-tri-Cl | H | |
| 19 | H | H | 2,4,6-tri-Br | H | |
| 20 | 4-F | H | H | H | |
| 21 | 4-F | H | 2-F | H | 111-118 |
| 22 | 4-F | H | 2-F | H (Isomer A) | 128-130 |
| 23 | 4-F | H | 2-F | H (Isomer B) | 76-78 |
| 24 | 4-F | H | 4-F | H | 145-148 |
| 25 | 4-F | H | 2,4-di-F | H | 132-138 |
| 26 | 4-F | H | 2-Cl | H | |
| 27 | 4-F | H | 4-Cl | H | |
| 28 | 4-F | H | 2,4-di-Cl | H | |
| 29 | 4-F | H | 2-Br | H | |
| 30 | 4-F | H | 4-Br | H | |
| 31 | 4-F | H | 2,4-di-Br | H | |
| 32 | 4-F | H | 3-F | H | |
| 33 | 4-F | H | 3-Cl | H | |

TABLE 1 (continued)

| EX. NO. | X | X' | Y' | Y | (m.p.°C) |
|---|---|---|---|---|---|
| 34 | 4-F | H | 3-Br | H | |
| 35 | 4-F | H | 2-$CF_3$ | H | |
| 36 | 4-F | H | 3-$CF_3$ | H | |
| 37 | 4-F | H | 4-$CF_3$ | H | |
| 38 | 4-F | H | 2,4,6-tri-F | H | |
| 39 | 4-F | H | 2,4,6-tri-Cl | H | |
| 40 | 4-F | H | 2,4,6-tri-Br | H | |
| 41 | 2,4-di-F | H | H | H | |
| 42 | 2,4-di-F | H | 2-F | H | |
| 43 | 2,4-di-F | H | 4-F | H | |
| 44 | 2,4-di-F | H | 2,4-di-F | H | |
| 45 | 2,4-di-F | H | 2-Cl | H | |
| 46 | 2,4-di-F | H | 4-Cl | H | |
| 47 | 2,4-di-F | H | 2,4-di-Cl | H | |
| 48 | 2,4-di-F | H | 2-Br | H | |
| 49 | 2,4-di-F | H | 4-Br | H | |
| 50 | 2,4-di-F | H | 2,4-di-Br | H | |
| 51 | 2-F | H | H | H | |
| 52 | 2-F | H | 2-F | H | |
| 53 | 2-F | H | 4-F | H | |
| 54 | 2-F | H | 2,4-di-F | H | |
| 55 | 2-F | H | 2-Cl | H | |
| 56 | 2-F | H | 4-Cl | H | |
| 57 | 2-F | H | 2,4-di-Cl | H | |
| 58 | 2-F | H | 2-Br | H | |
| 59 | 2-F | H | 4-Br | H | |
| 60 | 2-F | H | 2,4-di-Br | H | |
| 61 | 2,3-di-F | H | 4-F | H | |
| 62 | 2,3-di-F | H | 4-F | H | |
| 63 | 2,3-di-F | H | 4-F | H | |
| 64 | 2,3-di-F | H | 4-F | H | |
| 65 | 2,3-di-F | H | 4-F | H | |
| 66 | 2,3-di-Cl | H | 4-F | H | |
| 67 | 2,3-di-Cl | H | 4-F | H | |
| 68 | 2,3-di-Cl | H | 4-F | H | |
| 69 | 2,3-di-Cl | H | 4-F | H | |
| 70 | 2,3-di-Cl | H | 4-F | H | |
| 71 | 3-F | H | H | H | |
| 72 | 3-F | H | 2-F | H | |
| 73 | 3-F | H | 4-F | H | |
| 74 | 3-F | H | 2,4-di-F | H | |
| 75 | 3-F | H | 2-Cl | H | |

TABLE 1 (continued)

| EX. NO. | X | X' | Y' | Y | (m.p.°C) |
|---|---|---|---|---|---|
| 76 | 3-F | H | 4-Cl | H | |
| 77 | 3-F | H | 2,4-di-Cl | H | |
| 78 | 3-F | H | 2-Br | H | |
| 79 | 3-F | H | 4-Br | H | |
| 80 | 3-F | H | 2,4-di-Br | H | |
| 81 | 2-Cl | H | H | H | |
| 82 | 2-Cl | H | 2-F | H | |
| 83 | 2-Cl | H | 4-F | H | |
| 84 | 2-Cl | H | 2,4-di-F | H | |
| 85 | 2-Cl | H | 2-Cl | H | |
| 86 | 2-Cl | H | 4-Cl | H | |
| 87 | 2-Cl | H | 2,4-di-Cl | H | |
| 88 | 2-Cl | H | 2-Br | H | |
| 89 | 2-Cl | H | 4-Br | H | |
| 90 | 2-Cl | H | 2,4-di-Br | H | |
| 91 | 3-Cl | H | H | H | |
| 92 | 3-Cl | H | 2-F | H | |
| 93 | 3-Cl | H | 4-F | H | |
| 94 | 3-Cl | H | 2,4-di-F | H | |
| 95 | 3-Cl | H | 2-Cl | H | |
| 96 | 3-Cl | H | 4-Cl | H | |
| 97 | 3-Cl | H | 2,4-di-Cl | H | |
| 98 | 3-Cl | H | 2-Br | H | |
| 99 | 3-Cl | H | 4-Br | H | |
| 100 | 3-Cl | H | 2,4-di-Br | H | |
| 101 | 4-Cl | H | H | H | |
| 102 | 4-Cl | H | 2-F | H | |
| 103 | 4-Cl | H | 4-F | H | |
| 104 | 4-Cl | H | 2,4-di-F | H | |
| 105 | 4-Cl | H | 2-Cl | H | |
| 106 | 4-Cl | H | 4-Cl | H | |
| 107 | 4-Cl | H | 2,4-di-Cl | H | |
| 108 | 4-Cl | H | 2-Br | H | |
| 109 | 4-Cl | H | 4-Br | H | |
| 110 | 4-Cl | H | 2,4-di-Br | H | |
| 111 | 2,4-di-Cl | H | H | H | |
| 112 | 2,4-di-Cl | H | 2-F | H | |
| 113 | 2,4-di-Cl | H | 4-F | H | |
| 114 | 2,4-di-Cl | H | 2,4-di-F | H | |
| 115 | 2,4-di-Cl | H | 2-Cl | H | |
| 116 | 2,4-di-Cl | H | 4-Cl | H | |
| 117 | 2,4-di-Cl | H | 2,4-di-Cl | H | |
| 118 | 2,4-di-Cl | H | 2-Br | H | |
| 119 | 2,4-di-Cl | H | 4-Br | H | |
| 120 | 2,4-di-Cl | H | 2,4-di-Br | H | |
| 121 | 2-$CF_3$ | H | H | H | |

TABLE 1 (continued)

| EX. NO. | X | X' | Y' | Y | (m.p.°C) |
|---|---|---|---|---|---|
| 122 | $2-CF_3$ | H | 2-F | H | |
| 123 | $2-CF_3$ | H | 4-F | H | |
| 124 | $2-CF_3$ | H | 2,4-di-F | H | |
| 125 | $2-CF_3$ | H | 2-Cl | H | |
| 126 | $2-CF_3$ | H | 4-Cl | H | |
| 127 | $2-CF_3$ | H | 2,4-di-Cl | H | |
| 128 | $2-CF_3$ | H | 2-Br | H | |
| 129 | $2-CF_3$ | H | 4-Br | H | |
| 130 | $2-CF_3$ | H | 2,4-di-Br | H | |
| 131 | $3-CF_3$ | H | H | H | |
| 132 | $3-CF_3$ | H | 2-F | H | |
| 133 | $3-CF_3$ | H | 4-F | H | |
| 134 | $3-CF_3$ | H | 2,4-di-F | H | |
| 135 | $3-CF_3$ | H | 2-Cl | H | |
| 136 | $3-CF_3$ | H | 4-Cl | H | |
| 137 | $3-CF_3$ | H | 2,4-di-Cl | H | |
| 138 | $3-CF_3$ | H | 2-Br | H | |
| 139 | $3-CF_3$ | H | 4-Br | H | |
| 140 | $3-CF_3$ | H | 2,4-di-Br | H | |
| 141 | $4-CF_3$ | H | H | H | |
| 142 | $4-CF_3$ | H | 2-F | H | |
| 143 | $4-CF_3$ | H | 4-F | H | |
| 144 | $4-CF_3$ | H | 2,4-di-F | H | |
| 145 | $4-CF_3$ | H | 2-Cl | H | |
| 146 | $4-CF_3$ | H | 4-Cl | H | |
| 147 | $4-CF_3$ | H | 2,4-di-Cl | H | |
| 148 | $4-CF_3$ | H | 2-Br | H | |
| 149 | $4-CF_3$ | H | 4-Br | H | |
| 150 | $4-CF_3$ | H | 2,4-di-Br | H | |
| 151 | 3-Br | H | H | H | |
| 152 | 3-Br | H | 2-F | H | |

TABLE 1 (continued)

| EX. NO. | X | X' | Y' | Y | (m.p.°C) |
|---|---|---|---|---|---|
| 153 | 3-Br | H | 4-F | H | |
| 154 | 3-Br | H | 2,4-di-F | H | |
| 155 | 3-Br | H | 2-Cl | H | |
| 156 | 3-Br | H | 4-Cl | H | |
| 157 | 3-Br | H | 2,4-di-Cl | H | |
| 158 | 3-Br | H | 2-Br | H | |
| 159 | 3-Br | H | 4-Br | H | |
| 160 | 3-Br | H | 2,4-di-Br | H | |
| 161 | 4-Br | H | H | H | |
| 162 | 4-Br | H | 2-F | H | |
| 163 | 4-Br | H | 4-F | H | |
| 164 | 4-Br | H | 2,4-di-F | H | |
| 165 | 4-Br | H | 2-Cl | H | |
| 166 | 4-Br | H | 4-Cl | H | |
| 167 | 4-Br | H | 2,4-di-Cl | H | |
| 168 | 4-Br | H | 2-Br | H | |
| 169 | 4-Br | H | 4-Br | H | |
| 170 | 4-Br | H | 2,4-di-Br | H | |
| 171 | H | 4-$CH_3$ | H | H | |
| 172 | H | 4-$CH_3$ | 2-F | H | |
| 173 | H | 4-$CH_3$ | 4-F | H | |
| 174 | H | 4-$CH_3$ | 2,4-di-F | H | |
| 175 | H | 4-$CH_3$ | 2-Cl | H | |
| 176 | H | 4-$CH_3$ | 4-Cl | H | |
| 177 | H | 4-$CH_3$ | 2,4-di-Cl | H | |
| 178 | H | 4-$CH_3$ | 2-Br | H | |
| 179 | H | 4-$CH_3$ | 4-Br | H | |
| 180 | H | 4-$CH_3$ | 2,4-di-Br | H | |
| 181 | H | 4-$OCH_3$ | H | H | |
| 182 | H | 4-$OCH_3$ | 2-F | H | |
| 183 | H | 4-$OCH_3$ | 4-F | H | |
| 184 | H | 4-$OCH_3$ | 2,4-di-F | H | |
| 185 | H | 4-$OCH_3$ | 2-Cl | H | |
| 186 | H | 4-$OCH_3$ | 4-Cl | H | |
| 187 | H | 4-$OCH_3$ | 2,4-di-Cl | H | |
| 188 | H | 4-$OCH_3$ | 2-Br | H | |
| 189 | H | 4-$OCH_3$ | 4-Br | H | |

TABLE 1 (continued)

| EX. NO. | X | X' | Y' | Y | (m.p.°C) |
|---|---|---|---|---|---|
| 190 | H | 4-$OCH_3$ | 2,4-di-Br | H | |
| 191 | H | 2-$CH_3$ | H | H | |
| 192 | H | 2-$CH_3$ | 2-F | H | |
| 193 | H | 2-$CH_3$ | 4-F | H | |
| 194 | H | 2-$CH_3$ | 2,4-di-F | H | |
| 195 | H | 2-$CH_3$ | 2-Cl | H | |
| 196 | H | 2-$CH_3$ | 4-Cl | H | |
| 197 | H | 2-$CH_3$ | 2,4-di-Cl | H | |
| 198 | 4-F | 2-$CH_3$ | H | H | |
| 199 | 4-F | 2-$CH_3$ | 2-F | H | |
| 200 | 4-F | 2-$CH_3$ | 4-F | H | |
| 201 | 4-F | 2-$CH_3$ | 2,4-di-F | H | |
| 202 | 4-F | 2-$CH_3$ | 2-Cl | H | |
| 203 | 4-F | 2-$CH_3$ | 4-Cl | H | |
| 204 | 4-F | 2-$CH_3$ | 2,4-di-Cl | H | |
| 205 | 2-F | 4-$CF_3$ | H | H | |
| 206 | 2-F | 4-$CF_3$ | 2-F | H | |
| 207 | 2-F | 4-$CF_3$ | 4-F | H | |
| 208 | 2-F | 4-$CF_3$ | 2,4-di-F | H | |
| 209 | 2-F | 4-$CF_3$ | 2-Cl | H | |
| 210 | 2-F | 4-$CF_3$ | 4-Cl | H | |
| 211 | 2-F | 4-$CF_3$ | 2,4-di-Cl | H | |
| 212 | H | 4-$OCF_2H$ | H | H | |
| 213 | H | 4-$OCF_2H$ | 2-F | H | |
| 214 | H | 4-$OCF_2H$ | 4-F | H | |
| 215 | H | 4-$OCF_2H$ | 2,4-di-F | H | |
| 216 | H | 4-$OCF_2H$ | 2-Cl | H | |
| 217 | H | 4-$OCF_2H$ | 4-Cl | H | |
| 218 | H | 4-$OCF_2H$ | 2,4-di-Cl | H | |
| 219 | 4-F | 2-$OCH_3$ | H | H | |
| 220 | 4-F | 2-$OCH_3$ | 2-F | H | |

TABLE 1 (continued)

| EX. NO. | X | X' | Y' | Y | (m.p.°C) |
|---|---|---|---|---|---|
| 221 | 4-F | 2-$OCH_3$ | 4-F | H | |
| 222 | 4-F | 2-$OCH_3$ | 2,4-di-F | H | |
| 223 | 4-F | 2-$OCH_3$ | 2-Cl | H | |
| 224 | 4-F | 2-$OCH_3$ | 4-Cl | H | |
| 225 | 4-F | 2-$OCH_3$ | 2,4-di-Cl | H | |
| 226 | H | 4-$SCH_3$ | H | H | |
| 227 | H | 4-$SCH_3$ | 2-F | H | |
| 228 | H | 4-$SCH_3$ | 4-F | H | |
| 229 | H | 4-$SCH_3$ | 2,4-di-F | H | |
| 230 | H | 4-$SCH_3$ | 2-Cl | H | |
| 231 | H | 4-$SCH_3$ | 4-Cl | H | |
| 232 | H | 4-$SCH_3$ | 2,4-di-Cl | H | |
| 233 | 4-F | H | H | 2-$CH_3$ | |
| 234 | 4-F | H | 4-F | 2-$CH_3$ | |
| 235 | 4-F | H | 4-Cl | 2-$CH_3$ | |
| 236 | 4-F | H | 4-$CF_3$ | 2-$CH_3$ | |
| 237 | 4-F | H | 2-F | 4-$CH_3$ | |
| 238 | 4-F | H | 2-Cl | 4-$CH_3$ | |
| 239 | 4-F | H | 2-$CF_3$ | 4-$CH_3$ | |
| 240 | 4-F | H | 2-F | 4-$CF_3$ | |
| 241 | 4-F | H | 2-Cl | 4-$CF_3$ | |
| 242 | 4-F | H | 2-$CF_3$ | 4-$CF_3$ | |
| 243 | 4-F | H | H | 4-$OCF_2H$ | |
| 244 | 4-F | H | 2-F | 4-$OCF_2H$ | |
| 245 | 4-F | H | 2-Cl | 4-$OCF_2H$ | |
| 246 | 4-F | H | 2-$CF_3$ | 4-$OCF_2H$ | |
| 247 | 4-F | H | H | 4-$OCH_3$ | |
| 248 | 4-F | H | 2-Cl | 4-$OCH_3$ | |
| 249 | 4-F | H | 2-F | 4-$OCH_3$ | |
| 250 | 4-F | H | 2-$CF_3$ | 4-$OCH_3$ | |

TABLE 1 (continued)

| EX. NO. | X | X' | Y' | Y | (m.p.°C) |
|---|---|---|---|---|---|
| 251 | 4-F | H | H | 4-$SCH_3$ | |
| 252 | 4-F | H | 2-F | 4-$SCH_3$ | |
| 253 | 4-F | H | 2-Cl | 4-$SCH_3$ | |
| 254 | 4-F | H | 2-$CF_3$ | 4-$SCH_3$ | |

TABLE 2

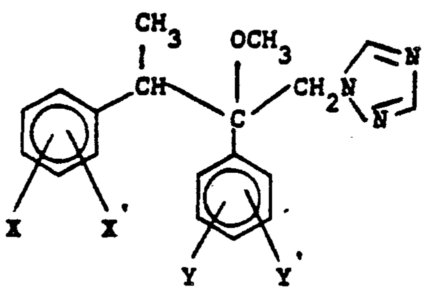

(I)

| EX. NO. | X | X' | Y' | Y | (m.p.°C) |
|---|---|---|---|---|---|
| 255 | H | H | 2-F | H | |
| 256 | H | H | 4-F | H | |
| 257 | H | H | 2,4-di-F | H | |
| 258 | H | H | 2-Cl | H | |
| 259 | H | H | 4-Cl | H | |
| 260 | H | H | 2,4-di-Cl | H | |
| 261 | 4-F | H | 2-F | H | |
| 262 | 4-F | H | 4-F | H | |
| 263 | 4-F | H | 2,4-di-F | H | |
| 264 | 4-F | H | 2-Cl | H | |
| 265 | 4-F | H | 4-Cl | H | |
| 266 | 4-F | H | 2,4-di-Cl | H | |

TABLE 3

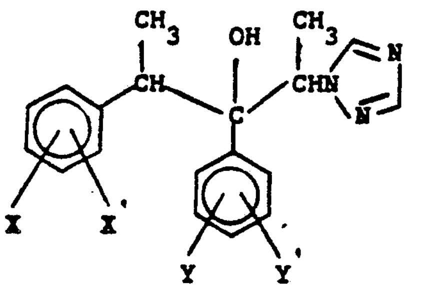

(I)

| EX. NO. | X | X' | Y' | Y | (m.p. °C) |
|---|---|---|---|---|---|
| 267 | H | H | 2-F | H | |
| 268 | H | H | 4-F | H | |
| 269 | H | H | 2,4-di-F | H | |
| 270 | H | H | 2-Cl | H | |
| 271 | H | H | 4-Cl | H | |
| 272 | H | H | 2,4-di-Cl | H | |
| 273 | 4-F | H | 2-F | H | |
| 274 | 4-F | H | 4-F | H | |
| 275 | 4-F | H | 2,4-di-F | H | |
| 276 | 4-F | H | 2-Cl | H | |
| 277 | 4-F | H | 4-Cl | H | |
| 278 | 4-F | H | 2,4-di-Cl | H | |

TABLE 4

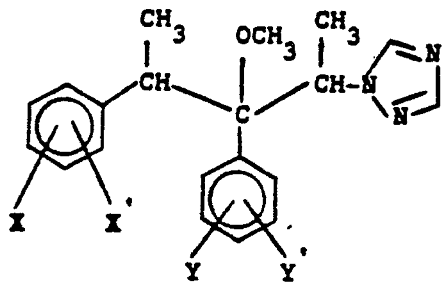

(I)

| EX. NO. | X | X' | Y' | Y | (m.p.°C) |
|---|---|---|---|---|---|
| 279 | H | H | 2-F | H | |
| 280 | H | H | 4-F | H | |
| 281 | H | H | 2,4-di-F | H | |
| 282 | H | H | 2-Cl | H | |
| 283 | H | H | 4-Cl | H | |
| 284 | H | H | 2,4-di-Cl | H | |
| 285 | 4-F | H | 2-F | H | |
| 286 | 4-F | H | 4-F | H | |
| 287 | 4-F | H | 2,4-di-F | H | |
| 288 | 4-F | H | 2-Cl | H | |
| 289 | 4-F | H | 4-Cl | H | |
| 290 | 4-F | H | 2,4-di-Cl | H | |

## Agricultural Formulations

The compounds of this invention when used for agricultural purposes will generally be used in formulation with a liquid or solid diluent or with an organic solvent. Useful formulations of the compounds of Formula I can be prepared in conventional ways. They include dusts, granules, pellets, solutions, emulsions, wettable powders, emulsifiable concentrates and the like. Many of these may be applied directly. Sprayable formulations can be extended in suitable media and used at spray volumes of from about one to several hundred liters per hectare. High strength compositions are primarily used as intermediates for further formulation. The formulations, broadly, contain about 1% to 99% by weight of active ingredient(s) and at least one of a) about 0.1% to 35% surfactant(s) and b) about 5% to 99% solid or liquid inert diluent(s). More specifically, they will contain these ingredients in the following approximate proportions:

| | Active Ingredient | Percent by Weight | |
|---|---|---|---|
| | | Diluent(s) | Surfactant(s) |
| Wettable Powders | 20-90 | 0-74 | 1-10 |
| Oil Suspensions, Emulsions, Solutions, (including Emulsifiable Concentrates) | 5-50 | 40-95 | 0-35 |
| Aqueous Suspensions | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey. The more absorptive diluents are preferred for the wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0° C. "McCutcheon's Detergents and Emulsifiers Annual", MC Publishing Corp., Ridgewood, New Jersey, as well as Sisely and Wood, "Encyclopedia of Surface Active Agents", Chemical Publ. Co., Inc., New York, 1964, list surfactants and recommended uses. All formulations can contain minor amounts of additives to reduce foam, caking, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon performed granular carriers or by agglomeration techniques. See J. E. Browning, "Agglomeration", Chemical Engineering, Dec. 4, 1967, pp. 147ff. and "Perry's Chemical Engineer's Handbook", 4th Edn., McGraw-Hill, N.Y., 1963, pp. 8-59ff.

For further information regarding the art of formulation, see for example:

H. M. Loux, U.S. Patent 3,235,361, Feb. 15, 1966, Col. 6, Line 16 through Col. 7, Line 19 and Examples 10 through 41.

R. W. Luckenbaugh, U.S. Patent 3,309,192, March 14, 1967, Col. 5, Line 43 through Col. 7, Line 62 and Examples 8, 12, 15, 39, 41, 52, 53, 58, 132, 138-140, 162-164, 166, 167, 169-182.

H. Gysin and E. Knusli, U.S. Patent 2,891,855, June 23, 1959, Col. 3, Line 66 through Col. 5, Line 17 and Examples 1-4.

G. C. Klingman, "Weed Control as a Science", John Wiley and Sons, Inc., New York, 1961, pp. 81-96.

J. D. Fryer and S. A. Evans, "Weed Control Handbook", 5th Edn. Blackwell Scientific Publications, Oxford, 1968, pp. 101-103.

Examples of useful formulations of compounds of the present invention are as follows:

Wettable Powder

| | |
|---|---|
| 2-(2,4-difluorophenyl)-3-(4-fluyorophenyl)-1-(1,2,4-triazol-1-yl)-3-butan-2-ol; and the (S) enantiomer thereof | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled, reblended and packaged.

Granule

| | |
|---|---|
| wettable powder of above example | 15% |
| gypsum | 69% |
| potassium sulfate | 16% |

The ingredients are blended in a rotating or fluid bed mixer and water sprayed on to accomplish granulation. When most of the material has reached the desired range of 1.0 to 0.42 mm. (U.S.S. No. 18 to 40 sieves), the granules are removed, dried, and screened. Oversize material is crushed to produce additional material in the desired range.

High Strength Concentrate

| | |
|---|---|
| 2-(2-fluorophenyl)-3-(4-fluorophenyl)-1-(1,2,4-triazol-1-yl)-3-butan-2-ol; and the (S) enantiomer thereof | 98.5% |
| silica aerogel | 0.5% |
| synthetic amorphous fine silica | 1.0% |

The ingredients are blended and ground in a hammer-mill to produce a high strength concentrate essentially all passing a U.S.S. No. 50 sieve (0.3 mm openings). This material may then be formulated in a variety of ways.

Aqueous Suspension

| | |
|---|---|
| 2-(2,4-difluorophenyl)-3-phenyl-1-(1,2,4-triazol-1-yl)-3-butan-2-ol; and the (S) enantiomer thereof | 25% |
| hydrated attapulgite | 3% |
| crude calcium ligninsulfonate | 20% |
| sodium dihydrogen phosphate | 0.5% |
| water | 61.5% |

The ingredients are ground together in a ball, sand, or roller mill until the solid particles have been reduced to diameters under 10 microns.

Solution

| | |
|---|---|
| 2-(2,4-difluorophenyl)-3-(4-chlorophenyl)-1-(1,2,4-triazol-1-yl)-3-butan-2-ol; and the (S) enantiomer thereof | 30% |
| dimethylformamide | 70% |

The ingredients are combined and stirred to produce a solution, which can be used for low volume applications.

Emulsifiable Concentrate

| | |
|---|---|
| 2-(2,4-difluorophenyl)-3-(4-fluorophenyl)-1-(1,2,4-triazol-1-yl)-3-butan-2-ol; and the (S) enantiomer thereof | 15% |
| blend of calcium sulfonates and nonionic surfactants | 25% |
| xylene | 60% |

The ingredients are combined and stirred until the active is dissolved. A fine screen filter is included in packaging operation to insure the absence of any extraneous undissolved material in the product.

Utility

The compounds of this invention are useful as plant disease control agnets. They are effective in controlling a broad spectrum of plant diseases, particularly foliar pathogens of ornamental, vegetable, field, cereal and fruit crops, such as Puccinia recondita, Erysiphe cichoracearum, Erysiphe graminis, Venturia

inaequalis, Cercospora arachidicola, and Monilinia fructicola, Rhizoctonia solani, Pyricularia oryzae, Botrytis cinerea, Pseudocercosporella herpotrichlorides, and Cercosporidium personatum. They also control seed pathogens.

Disease control is ordinarily accomplished by applying an effective amount of the compound either pre- or post-infection to the portion of the plant to be protected, such as the roots, stems, foliage, fruit, seeds, tubers or bulbs, or to the media (soil or sand) in which the plants to be protected are growing. The compound may also be applied to the seed from which the plants to be protected are to be grown.

Rates of application for these compounds can be influenced by many factors of the environment and should be determined under actual use conditions. Foliage can normally be protected when treated at a rate of from less than 1 g/ha to 5000 g/ha of active ingredient. Plants growing in soil treated at a concentration from 0.1 to about 20 kg/ha can be protected from disease. Seed and seedlings can normally be protected when seed is treated at a rate of from 0.06 to about 3 grams per kilogram of seed.

The compounds of this invention can be mixed with fungicides, bactericides, acaricides, nematicides, insecticides, or other biologically active compounds in order to achieve desired results with a minimum expenditure of time, effort and material. Amounts of these biologically active materials added for each part by weight of the composition of this invention may vary from 0.05 to 25 parts by weight. Suitable agents of this type are well-known to those skilled in the art, Some are listed below:

## Fungicides

methyl 2-benzimidazolecarbamate (carbendazim)
tetramethylthiuram disulfide (thiuram)
n-dodecylguanidine acetate (dodine)
manganese ethylenebisdithiocarbamate (maneb)
1,4-dichloro-2,5-dimethoxybenzene (chloroneb)methyl
1-(butylcarbamoyl)-2-benzimidazolecarbamate (benomyl)
2-cyano-N-ethylcarbamoyl-2-methoxyiminoacetamide (cymoxanil)
N-trichloromethylthiotetrahydrophthalamide (captan)
N-trichloromethylthiophthalimide (folpet)
dimethyl 4,4′-(o-phenylene)bis(3-thioallophanate)-(thiophanate-methyl)
2-(thiazol-4-yl)benzimidazole (thiabendazole)
aluminum tris(O-ethyl phosphonate) (phosethyl aluminum)
tetrachloroisophthalonitrile (chlorothalonil)
2,6-dichloro-4-nitroaniline (dichloran)
N-(2,6-dimethylphenyl)-N-(methoxyacetyl)alanine methyl ester (metalaxyl)
cis-N-[1,1,2,2-tetrachloroethyl)thio]cyclohex-4-ene-1,2-dicarbioximide (captafol)
3-(3,5-dichlorophenyl)-N-(1-methylethyl)-2,4-dioxo-1-imidazolidine carboxamide (iprodione)
3-(3,5-dichlorophenyl)-5-ethenyl-5-methyl-2,4-oxazolidinedione (vinclozolin)
kasugamycin
O-ethyl-S,S-diphenylphosphorodithioate (edifenphos)
4-(3-(4-(1,1-dimethyl-ethyl)phenyl-2-methyl)propyl-2,6-dimethylmorpholine (Fenpropimorph)
4-(3-(4-1,1-dimethyl-ethyl)phenyl-2-methyl)propylpyridine (Fenpropidin)

## Bactericides

tribasic copper sulfate
streptomycin sulfate
oxytetracycline

## Acaricides

senecioic acid, ester with 2-sec-butyl-4,6-dinitrophenol (binapacryl)

6-methyl-1,3-dithiolo[2,3-B]quinonolin-2-one (oxythioquinox)
2,2,2-trichloro-1,1-bis(4-chlorophenyl)ethanol-(dicofol)
bis(pentachloro-2,4-cyclopentadien-1-yl) (dienochlor)
tricyclohexyltin hydroxide (cyhexatin)
hexakis(2-methyl-2-phenylpropyl)distannoxane (fenbutin oxide)

## Nematicides

2-[diethoxyphosphinylimino]-1,3-diethietane (fosthietan)
S-methyl-1-(dimethylcarbamoyl)-N-(methylcarbamoyloxy)-thioformimidate (oxamyl)
S-methyl-1-carbamoyl-N-(methylcarbamoyloxy)thioformimidate
N-isopropylphosphoramidic acid, O-ethyl-O′-]4-(methylthio)-m-tolyl]diester (fenamiphos)

## Insecticides

3-hydroxy-N-methylcrotonamide(dimethylphosphate)ester (monocrotophos)
methylcarbamic acid, ester with 2,3-dihydro-2,2-dimethyl-7-benzofuranol (carbofuran)
O-[2,4,5-trichloro-α-(chloromethyl)benzyl]phosphoric acid, O′, O′-dimethyl ester (tetrachlorvinphos)
2-mercaptosuccinic acid, diethyl ester, S-ester with thionophosphoric acid, dimethyl ester (malathion)
phosphorothioic acid, O,O-dimethyl, O-p-nitrophenyl ester (methyl parathion)
methylcarbamic acid, ester with α-naphthol (carbaryl)
methyl N-[[(methylamino)carbonyl]oxy]ethanimidothioate (methomyl)
N′-(4-chloro-o-tolyl)-N,N-dimethylformamidine (chlordimeform)
O,O-diethyl-O-(2-isopropyl-4-methyl-6-pyrimidyl)-phosphorothioate (diazinon)
octachlorocamphene (toxaphene)
O-ethyl O-p-nitrophenyl phenylphosphonothioate (EPN)
cyano(3-phenoxyphenyl)-methyl 4-chloro-α-(1-methylethyl)benzeneacetate (fenvalerate)
(3-phenoxyphenyl)methyl (±)-cis, trans-3-(2,2-dichloroethenyl)-2,2-dimethylcyclopropanecarboxylate (permethrin)
dimethyl N,N′[thiobis(N-methylimmo)carbonyloxy]]bis[ethanimidothioate] (thiodicarb)
phosphorothiolothionic acid, O-ethyl-O-[4-(methylthio)pehnyl]-S-n-propyl ester (sulprofos)
α-cyano-3-phenoxybenzyl 3-(2,2-dichlorovinyl)-2,2-dimethylcyclopropane carboxylate (cypermethrin)
cyano(3-phenoxyphenyl)methyl 4-(difluoromethoxy)-α-(methylethyl)benzeneacetate (flucythrinate)
O,O-diethyl-O-(3,5,6-trichloro-2-pyridyl)phosphorothioate (chlorpyrifos)
O,O-dimethyl-S-[4-oxo-1,2,3-benzotriazin-3-(4H)-yl)methyl]phosphorodithioate (azinphos-methyl)
5,6-dimethyl-2-dimethylamino-4-pyrimidinyl dimethyl carbamate (pirimicarb)
S-(N-formyl-N-methylcarbamoylmethyl)-O,O-dimethyl phosphorodithioate (formothion)
S-2-(ethylthioethyl)-O,O-dimethyl phosphiorothioate (demeton-S-methyl)
α-cyano-3-phenoxybenzyl cis-3-(2,2-dibromovinyl)-2,2-dimethylcyclopropane carboxylate (deltamethrin)
cyano(3-phenoxyphenyl)methyl ester of N-(2-chloro-4-trifluoromethylphenyl)alanine (fluvalinate)

This invention is further illustrated by the following examples.

## Example A

The test compounds were dissolved in acetone in an amount equal to 6% of the final volume and then suspended at a concentration of 100 or 40 ppm in purified water containing 250 ppm of the surfactant TREM 014 (polyhydric alcohol esters). This suspension was sprayed to the point of run-off on apple seedlings. The following day plants were inoculated with a spore suspension of *Venturia inaequalis*, the causal agent of apple scab, and incubated in a saturated humidity chamber at 20°C for 24 hours and then in a growth chamber at 22°C for 11 days, when disease ratings were made.

Example B

The test compounds were dissolved in acetone in an amount equal to 6% of the final volume and then suspended at a concentration of 100 or 40 ppm in purified water containing 250 ppm of the surfactant TREM 014 (polyhydric alcohol esters). This suspension was sprayed to the point of run-off on peanut seedlings. The following day plants were inoculated with a spore suspension of Cercosporidium personatum, the causal agent of Peanut Late Leafspot, and incubated in a saturated himidity chamber at 22° C for 24 hours, then in a high himidity chamber at 27° C for 7 days, and then in a growth chamber at 29° C for 7 days, when disease ratings were made.

Example C

The test compounds were dissolved in acetone in an amoutn equal to 6% of the final volume and then suspended at a concentration of 100 or 40 ppm in purified water containing 250 ppm of the surfactant TREM 014 (polyhydric alcohol esters). This suspension was sprayed to the point of run-off on broad bean seedlings. The following day plants were inoculated with a spore suspension of Botrytis cinerea, the causal agent of bean grey mold, and incubated in a saturated himidity chamber at 20° C for 24 hours when disease ratings were made.

Example D

The test compounds were dissolved in acetone in an amoutn equal to 6% of the final volume and then suspended at a concentration of 100 or 40 ppm in purified water containing 250 ppm of the surfactant TREM 014 (polyhydric alcohol esters). This suspension was sprayed to the point of run-off on wheat seedlings. The following day plants were inoculated with a spore dust of Erysiphe graminis f. sp. tritici, the causal agent of wheat powdery mildew, and incubated in a growth chamber at 20° C for 6 days, when disease ratings were made.

Example E

The test compounds were dissolved in acetone in an amount equal to 6% of the final volume and then suspended at a concentration of 100 or 40 ppm in purified water containing 2509 ppm of the surfactant TREM 014 (polyhydric alcohol esters). This suspension was sprayed to the point of run-off on rice seedlings. The following day plants were inoculated with a spore suspension of Pyricularia oryzae. the causal agent of rice blast, and incubated in a saturated himidity chamber at 27° C for 24 hours and then in a growth chamber at 29° C for 4 days, when disease ratings were made.

Example F

The test compounds were dissolved in acetone in an amount equal to 6% of the final volume and then suspended at a concentration of 100 or 40 ppm in purified water containing 250 ppm of the surfactant TREM 014 (polyhydric alcohol esters). This suspension was sprayed to the point of run-off on rice seedlings. The following day plants were inoculated with a spore suspension of Rhizoctonia solani, the causal agent of rice sheath blight, and incubated in a saturated humidity chamber at 27° C for 48 hours and then in a growth chamber at 29° C for 4 days, when disease ratings were made.

Example G

The test compounds were dissolved in acetone in an amount equal to 6% of the final volume and then suspended at a concentration of 100 or 40 ppm in purified water containing 250 ppm of the surfactant TREM 014 (polyhydric alcohol esters). This suspension was sprayed to the point of run-off on wheat seedlings. The following day plants were inoculated with a spore suspension of Puocinia recondita, the causal agent of wheat leaf rust, and incubated in a saturated humidity chamber at 20° C for 24 hours and then in a growth chamber at 20° C for 8 days, when disease ratings were made.

## Example H

The test compounds were dissolved in acetone in an amount equal to 6% of the final volume and then suspended at a concentration of 100 or 40 ppm in purified water containing 250 ppm of the surfactant TREM 014 (polyhydric alcohol esters). This suspension was sprayed to the point of run-off on tomato seedlings. The following day plants were inoculated with a spore suspension of Phytophthora infestans, the causal agent of tomato late blight, and incubated in a saturated humidity chamber at 20° C for 24 hours and then in a growth chamber at 20° C for 5 days, when disease ratings were made.

## Example I

The test compounds were dissolved in acetone in an amount equal to 6% of the final volume and then suspended at a concentration of 100 or 40 ppm in purified water containing 250 ppm of the surfactant TREM 014 (polyhydric alcohol esters). This suspension was sprayed to the point of run-off on grape seedlings. The following day plants were inoculated with a spore suspension of Plasmopara victicola, the causal agent of grade downy mildew, and incubated in a saturated humidity chamber at 20° C for 24 hours and then in a growth chamber at 20° C for 7 days, and then held in a saturated humidity chamber at 20° C for 24 hours, when disease ratings were made.

## Example J

The test compounds were dissolved in acetone 3% and water so that 1 ml of solution yielded a concentration of 5 kilogram/hectare when added to cucumber seeds and soil in pots. Seeds and soil were then inoculated with a mixture of sand, cereal and mycelium of the fungus Pythium aphanadermatum, causal agent of cucumber damping off, and incubated in a growth chamber at 30° C for 14 days. Disease ratings were then made.

## Example K

The test compounds were dissolved in acetone 3% and water so that 1 ml of solution yielded a concentration of 5 kilogram/hectare when added to cotton seeds and soil in pots. Seeds and soil were then inoculated with a mixture of sand, cereal and mycelium of the fungus Rhizoctonia solani, causal agents of cotton blight, and incubated in a growth chamber at 30° C for 14 days. Disease ratings were then made.

## Example L

The test compounds were dissolved in acetone 3% and water so that 1 ml of solution yielded a concentration of 5 kilogram/hectare when added to cucumber seeds and soil in pots. Seeds and soil were then inoculated with a mixture of sand, cereal and mycelium of the fungus Fusarium oxysporum f. sp. cucumerinum, causal agent of cucumber wilt, and incubated in a growth chamber at 30° C for 14 days. Disease ratings were then made.

## Example M

The test compounds were dissolved in acetone 3% and water so that 1 ml of solution yielded a concentration of 5 kilogram/hectare when added to lima bean seeds and soil in pots. Seeds and soil were then inoculated with a mixture of sand, cereal and mycelium of the fungus Sclerotium rolfsii, causal agent of southern blight, and incubated in a growth chamber at 30° C for 14 days. Disease ratings were then made.

Results of Examples A-M are given in Table 5. In this table, a rating of 100 indicates 100% disease control and a rating of 0 indicates no disease control relative to the controls. A - entry indicates that no test was performed with the specified compound. A P entry indicates that disease control was not measured due to phytoxicity.

Examples J, K, L and M are conducted at the 5 kg/ha rate in all cases where results are shown. Application rate for Examples A to I are as indicated in the table.

TABLE 5

| EX. NO. | RATE (PPM) | EX. A | EX. B | EX. C | EX. D | EX. E | EX. F | EX. G | EX. H | EX. I | EX. J | EX. K | EX. L | EX. M |
|---|---|---|---|---|---|---|---|---|---|---|---|---|---|---|
| 25 | 40 | 100 | 100 | 98 | 100 | 93 | 97 | 100 | 0 | 0 | 0 | 60 | 0 | 0 |
| 21 | 40 | 100 | 100 | 98 | 100 | 84 | 93 | 100 | 0 | 23 | 0 | 50 | 0 | 50 |
| 4 | 100 | 100 | 100 | 96 | 99 | 78 | 71 | 100 | 18 | 100 | 0 | 80 | 100 | 0 |
| | 40 | 85 | 100 | 94 | 100 | 0 | 71 | 100 | 0 | 0 | - | - | - | - |
| 6 | 100 | 100 | 100 | 99 | 100 | 37 | 81 | 100 | 0 | 0 | 0 | 0 | 50 | 0 |
| | 40 | 100 | - | 81 | 99 | 37 | 62 | 100 | 0 | 0 | - | - | - | - |
| 24 | 100 | 100 | 100 | 90 | 100 | 97 | 93 | 100 | 0 | 0 | 0 | 90 | 50 | 100 |

## Claims

1. A compound of the formula

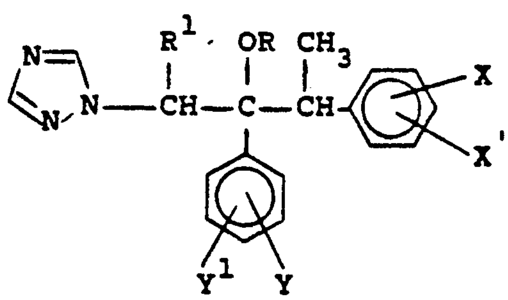

wherein:
R and $R^1$ are independently H or $CH_3$;
X and $Y^1$ are independently H, 1 to 3 halogens or $CF_3$; and
$X'$ and Y are independently H, $CH_3$, $CF_3$, $OCF_2H$, $OCH_3$ or $SCH_3$
and their agriculturally and pharmaceutically suitable salts.

2. Compounds of Claim 1 wherein R and $R^1$ are H.

3. Compounds of Claim 1 wherein X and $Y^1$ are independently 1 to 2 F, Cl, or Br.

4. Compounds of Claim 1 wherein
X and $Y^1$ are independently 1 to 2 F or Cl; and $X^1$ and Y are H.

5. Compounds of Claim 1 which are
1-(1,2,4-triazol-1-yl)2-(4-fluorophenyl)-3-phenyl butane-2-ol;
1-(1,2,4-triazol-1-yl)2-(2,4-difluorophenyl)-3-phenylbutane-2-ol;

1-(1,2,4-triazol-1-yl)2-(4-fluorophenyl)-3-(4-fluorophenyl)butane-2-ol;
1-(1,2,4-triazol-1-yl)2-(2-fluorophenyl)-3-(4-fluorophenyl)butane-2-ol;
1-(1,2,4-triazol-1-yl)2-(2,4-difluorophenyl)-3-(4-fluorophenyl)butane-2-ol;
1-(1,2,4-triazol-1-yl)2-(2-chlorophenyl)-3-(4-fluorophenyl)butane-2-ol;
1-(1,2,4-triazol-1-yl)2-phenyl-3-(4-fluorophenyl)butane-2-ol;
1-(1,2,4-triazol-1-yl)2-(2,4-difluorophenyl)-3-(4-chlorophenyl)butane-2-ol;
1-(1,2,4-triazol-1-yl)2-(2,4-difluorophenyl)-3-(2-chlorophenyl)butane-2-ol;
1-(1,2,4-triazol-1-yl)2-(2-fluorophenyl)-3-(3-chlorophenyl)butane-2-ol;
1-(1,2,4-triazol-1-yl)2-(2,4-difluorophenyl)-3-(3-chlorophenyl)butane-2-ol;
1-(1,2,4-triazol-1-yl)2-(2,4-difluorophenyl)-3-(3-fluorophenyl)butane-2-ol;
1-(1,2,4-triazol-1-yl)2-(2-fluorophenyl)-3-(2,4-dichlorophenyl)butane-2-ol;
1-(1,2,4-triazol-1-yl)2-(2,4-difluorophenyl)-3-(2,4-dichlorophenyl)butane-2-ol;
1-(1,2,4-triazol-1-yl)2-(2,4-dichlorophenyl)-3-(2,4-dichlorophenyl)butane-2-ol;
1-(1,2,4-triazol-1-yl)2-(2-fluorophenyl)-3-(2,4-difluorophenyl)butane-2-ol;
1-(1,2,4-triazol-1-yl)2-(2,4-difluorophenyl)-3-(2,4-difluorophenyl)butane-2-ol;
1-(1,2,4-triazol-1-yl)2-(2,4-difluorophenyl)-3-(2-fluorophenyl)butane-2-ol;
1-(1,2,4-triazol-1-yl)2-(4-chlorophenyl)-3-(2-fluorophenyl)butane-2-ol;
and their agriculturally and pharmaceutically suitable salts.

6. An agricultural composition for controlling a plant fungus disease which comprises an effective amount of a compound of Claim 1 and at least one of the following: surfactant, solid or liquid inert diluent.

7. An agricultural composition for controlling a plant fungus disease which comprises an effective amount of a compound of Claim 2 and at least one of the following: surfactant, solid or liquid inert diluent.

8. An agricultural composition for controlling a plant fungus disease which comprises an effective amount of a compound of Claim 3 and at least one of the following: surfactant, solid or liquid inert diluent.

9. An agricultural composition for controlling a plant fungus disease which comprises an effective amount of a compound of Claim 4 and at least one of the following: surfactant, solid or liquid inert diluent.

10. An agricultural composition for controlling a plant fungus disease which comprises an effective amount of a compound of Claim 5 and at least one of the following: surfactant, solid or liquid inert diluent.

11. A method for controlling fungus disease in a plant comprising applying to the locus of infestation to be protected an effective amount of a compound of Claim 1.

12. A method for controlling fungus disease in a plant comprising applying to the locus of infestation to be protected an effective amount of a compound of Claim 2.

13. A method for controlling fungus disease in a plant comprising applying to the locus of infestation to be protected an effective amount of a compound of Claim 3.

14. A method for controlling fungus disease in a plant comprising applying to the locus of infestation to be protected an effective amount of a compound of Claim 4.

15. A method for controlling fungus disease in a plant comprising applying to the locus of infestation to be protected an effective amount of a compound of Claim 5.

European Patent Office

# EUROPEAN SEARCH REPORT

Application number

EP 88311828.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A | EP - A2 - 0 251 086 (DU PONT)<br>* Pages 11,12; claims 21,22 *<br>-- | 1,6-15 | C 07 D 249/08<br>A 01 N 43/653 |
| A | EP - A2 - 0 137 717 (DU PONT)<br>* Page 14, line 27; page 15, line 25; claims 8,9 *<br>-- | 1,6-15 | |
| A | DE - A1 - 2 851 086 (BAYER)<br>* Page 11; compounds 2,3 *<br>-- | 1 | |
| D,A | EP - A1 - 0 015 756 (JCJ)<br>* Claims 1,10,11; table I *<br>-- | 1,6-15 | |
| A | AT - B - 371 451 (JCJ)<br>* Formula I, table *<br>---- | 1 | |

| TECHNICAL FIELDS SEARCHED (Int. Cl.4) |
|---|
| C 07 D 249/00 |

The present search report has been drawn up for all claims

| Place of search | Date of completion of the search | Examiner |
|---|---|---|
| VIENNA | 15-02-1989 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82